# EUROPEAN PATENT SPECIFICATION

(11) **EP 1 189 876 B1**
(45) Date of publication and mention of the grant of the patent: **25.08.2004**
(21) Application number: 00926337.7
(22) Date of filing: 25.04.2000
(51) Int. Cl.: C07C 253/00

(54) **PROCESS FOR HYDROGENATING DINITRILES IN AMINONITRILES**
VERFAHREN ZUR HYDRIERUNG VON DINITRILEN ZU AMINONITRILEN
PRODUCTION D'AMINONITRILE

(30) Priority: 28.04.1999 US 300507; 02.07.1999 US 347815; 02.07.1999 US 347817; 02.07.1999 US 347812; 30.11.1999 US 168035 P; 07.01.2000 US 174998 P; 08.03.2000 US 188289 P; 10.03.2000 US 188661 P; 10.03.2000 US 188664 P; 03.04.2000 US 194248 P
(43) Date of publication of application: 27.03.2002
(73) Proprietor: INVISTA Technologies S.à.r.l., Wilmington, DE 19805 (US)
(72) Inventor: IONKIN, Alex Sergey, Newark, DE 19702 (US); ZIEMECKI, Stanislaw, Bodgan, Wilmington, DE 19805 (US); HARPER, Mark, J., Lewes, DE 19958 (US); KOCH, Theodore, Augur, Wilmington, DE 19808 (US); BRYNDZA, Henry, Edward, Avondale, PA 19311 (US)
(74) Representative: Carpmaels & Ransford
(86) International application number: PCT/US2000/011045
(87) International publication number: WO 2000/064862

(56) References cited:
- DE-C- 19 747 913
- US-A- 4 472 529
- US-A- 4 499 204
- US-A- 5 296 628
- DATABASE WPI Section Ch, Week 199716 Derwent Publications Ltd., London, GB; Class A60, AN 1997-175657 XP002143882 & JP 09 040630 A (SHOWA DENKO KK), 10 February 1997 (1997-02-10)
- DATABASE WPI Section Ch, Week 199431 Derwent Publications Ltd., London, GB; Class D15, AN 1994-251855 XP002152566 & JP 06 182203 A (SAKAI KAGAKU KOGYO KK), 5 July 1994 (1994-07-05)
- DATABASE CHEMABS [Online] CHEMICAL ABSTRACTS SERVICE, COLUMBUS, OHIO, US; SZILAGYI, TIBOR: "Infrared spectra of methyl cyanide and methyl isocyanide adsorbed on platinum/silica" retrieved from STN Database accession no. 110:64225 XP002152565 & APPL. SURF. SCI. (1988), 35(1), 19-26 ,

## Description

### FIELD OF THE INVENTION

The invention relates to a selective hydrogenation process for producing aminonitriles from corresponding dinitriles in the presence of one or more additives that improve the yield of and/or selectivity to the aminonitrile.

### BACKGROUND OF THE INVENTION

Aminonitriles are a class of important chemicals that have a variety of industrial applications. For example, aminonitriles can be used as monomers for producing high molecular weight polyamides. Specifically, 6-aminocapronitrile can be used to produce nylon 6.

Aminonitriles can be produced by catalytic partial hydrogenation of dinitriles. See, for example, US2208598, US2257814, US2762835, US3322815, US3350439, US3591618, US4389348, US4601859, US5151543, US5296628, US5512697, US5527946, DE836938, DE848654, DE-A-19636768 and WO99/47492. However, the yield of and selectivity to a desired aminonitrile using some of the known processes may not be as high as desired, and the amount of the complete hydrogenation product (diamine) is also generally higher than desired.

WO99/47492 mentioned above describes the use of certain carbonyl group-containing compounds as additives in the partial hydrogenation process to improve the yield of and/or selectivity to the desired aminonitrile product, and/or reduce the amount of fully hydrogenated product (diamine) produced.

JP 9040630 discloses the selective hydrogenation and reduction of a nitrile group of an aromatic dinitrile in the presence of a Raney catalyst which has been pre-treated with a solvent and a gas.

We have now found new classes of compounds that also effectively function as improved yield and/or selectivity additives in the partial hydrogenation processes such as, for example, those mentioned in previously mentioned WO99/47492.

### SUMMARY OF THE INVENTION

In accordance with one aspect of the present invention, there is provided a process for the partial hydrogenation of a dinitrile to an aminonitrile, comprising the step of contacting the dinitrile with a hydrogen-containing fluid in the presence of (a) a solvent comprising liquid ammonia, an alcohol, or both; (b) a hydrogenation catalyst; and (c) an additive for improving the yield of and/or selectivity to the aminonitrile, characterized in that the dinitrile has the general formula R(CN)₂, wherein R is an alkylene group, and the additive is carbon monoxide.

An advantage of this invention is that an aminonitrile can be produced in higher yield and/or having a higher selectivity to the aminonitrile with the additive than without. Other objects and advantages will become more apparent as the invention is more fully disclosed herein below.

### DETAILED DESCRIPTION OF THE PREFERRED EMBODIMENTS

According to this invention, a dinitrile is contacted with a hydrogen-containing fluid in the presence of a solvent, a catalyst and an additive as generally described above.

Suitable dinitriles for use herein have the general formula R(CN)₂, wherein R is an alkylene group. One dinitrile or combinations of different dinitriles may be used. Preferred alkylene groups contain from 2 to 25, more preferably 2 to 15, and most preferably 2 to 10 carbon atoms per group. In other words, preferred dinitriles contain from 4 to 27, more preferably 4 to about 17, and most preferably 4 to 12, carbon atoms per dinitrile molecule.

Examples of suitable dinitriles include, but are not limited to, adiponitrile; methylglutaronitrile; alpha,omega-pentanedinitrile; alpha,omega-heptanedinitrile; alpha,omega-nonanedinitrile; alpha,omega-dodecanedinitrile; alpha,omega-pentadecanedinitrile; alpha,omega-icosanedinitrile; alpha,omega-tetracosane-dinitrile; 3-methylhexanedinitrile; 2-methyl-4-methylene-octanedinitrile; and combinations of two or more thereof.

Preferably the carbon atoms of the starting dinitrile are arranged in a branched or linear chain. Preferred examples are adiponitrile (hydrogenated to 6-aminocapronitrile), methylglutaronitrile (hydrogenated to two isomeric aminonitriles: 5-amino-2-methylvaleronitrile and 5-amino-4-methylvaleronitrile) and alpha,omega-dodecanedinitrile (hydrogenated to the corresponding aminonitrile). The preferred dinitrile is adiponitrile because its selective hydrogenation product, 6-aminocapronitrile, is a well-known monomer for polymerization applications.

Any hydrogen-containing fluid can be used in the invention as long as there is sufficient hydrogen in the fluid to selectively hydrogenate a dinitrile to an aminonitrile. The term "fluid" refers to liquid, gas or both. The hydrogen content in the fluid can range from 1 to 100%, preferably about 50 to about 100%, and most preferably 90 to 100% by volume. The presently preferred hydrogen-containing fluid is substantially pure hydrogen gas.

The molar ratio of hydrogen (in the hydrogen-containing fluid) to dinitrile is not critical as long as sufficient hydrogen is present to produce the desired aminonitrile. Hydrogen is generally used in excess. Hydrogen pressures are generally in the range of about 50 to about 2000 psig (about 0.45 to about 13.89 MPa), with from about 200 to about 1000 psig (about 1.48 to about 7.00 MPa) preferred.

It should be noted that, unless otherwise indicated, pressures expressed as "psi" are gauge pressures (e.g., psig), and pressures expressed as "MPa" are absolute pressures.

Any solvent that comprises either liquid ammonia or an alcohol can be used in the invention. The concentration of liquid ammonia in the solvent can range from about 20 to about 100%, preferably about 50 to about 100%, and most preferably about 80% to about 100%, by weight of total solvent. A substantially pure liquid ammonia is preferred. However, if an alcohol is also present in the solvent, the concentration of ammonia can be adjusted based on the quantity of alcohol used, which is discussed in further detail below. The molar ratio of ammonia to dinitrile is preferably about 1:1 or greater, and is generally in the range of from about 1:1 to about 30:1, more preferably from about 2:1 to about 20:1.

Any alcohol that can facilitate the selected hydrogenation of a dinitrile to an aminonitrile can be used in this invention. Preferred are alcohols with 1 to 10, more preferably 1 to 4, carbon atoms per molecule. Examples of suitable alcohols include, but are not limited to, methanol, ethanol, propanol, isopropyl alcohol, butanol, isobutyl alcohol, pentanol, hexanol, heptanol, octanol, nonanol, decanol, and combinations of two or more thereof. The most preferred alcohol (when used) is methanol. The alcohol can generally be present in the solvent in the concentration of from about 20 to about 100%, preferably about 30 to about 99%, by weight based on the total solvent weight.

Typically when an alcohol is use, the solvent further comprises a base that is substantially soluble in the solvent. The term "substantially" refers to "more than trivial". Preferred bases are ammonia, an ammonium base or an inorganic base such as, for example, alkali metal oxides, alkaline earth metal oxides, alkali metal hydroxides, alkaline earth metal hydroxides, partially neutralized acids in which one or more protons of the acids are replaced with ammonium ion, alkali metal ions, alkaline earth metal ions, or combinations of two or more thereof. Specific examples of suitable bases include, but are not limited to ammonia, lithium hydroxide, sodium hydroxide, potassium hydroxide, sodium bicarbonate, sodium carbonate, potassium bicarbonate, or combinations of two or more thereof. The most preferred bases are ammonia, lithium hydroxide and sodium hydroxide for they are readily available and inexpensive.

A base can be present in the solvent in any quantity so long as the quantity can facilitate the selective hydrogenation of a dinitrile to an aminonitrile. Generally, a base can be present in the solvent in the range of from about 0.1 to about 10 weight%, based on the total weight of the starting dinitrile.

The catalyst in the process is a hydrogenation catalyst suitable for hydrogenating a dinitrile to an aminonitrile. Preferred are catalysts based on transition metals selected from the group consisting of iron, cobalt, nickel, rhodium and combinations thereof. The catalyst may also contain one or more promoters in addition to the transition metals mentioned above, for example, one or more of Group VIB and Group VII metals such as chromium, molybdenum and tungsten. The catalyst can also be in the form of an alloy, including a solid solution of two or more metals, or an individual metal.

The catalytic metal can also be supported on an inorganic support such as alumina, magnesium oxide and combinations thereof. The metal can be supported on an inorganic support by any means known to one skilled in the art such as, for example, impregnation, coprecipitation, ion exchange, and combinations of two or more thereof. The preferred inorganic support is magnesium oxide, and the preferred supported catalyst is a magnesium oxide supported nickel-iron catalyst.

The catalyst can be present in any appropriate physical shape or form. It can be in fluidizable forms, extrudates, tablets, spheres or combinations of two or more thereof. The catalyst may be in sponge metal form, for example, the Raney® nickels and Raney® cobalts. The molar ratio of catalyst to dinitrile can be any ratio as long as the ratio can catalyze the selective hydrogenation of a dinitrile. The weight ratio of catalyst to dinitrile is generally in the range of from about 0.0001:1 to about 1:1, preferably about 0.001:1 to about 0.5:1. If the catalytic metal is supported on an inorganic support or is a portion of alloy or solid solution, the catalytic metal is generally present in the range of from about 0.1 to about 60, preferably about 1 to about 50, and most preferably about 2 to about 50 weight %, based on the total catalyst weight.

The preferred catalyst is a sponge metal type catalyst. The metallic component is iron, cobalt, nickel or combinations thereof. Commercially available catalysts of this type are promoted or unpromoted Raney® Ni or Raney® Co catalysts that can be obtained from the Grace Chemical Co. (Columbia, Maryland), or alternative sponge metal catalysts available, for example, from Activated Metals Corporation (Sevierville, Tenn.) or Degussa (Ridgefield Park, N.J.).

In the case of the supported nickel/iron catalyst, the rate of adiponitrile conversion increases with the amount of Ni deposited on the support. The preferred concentration of Ni is between about 5 and about 50 weight%, and especially between about 25 and about 35 weight%, based on the catalyst weight (metals + support). The preferred concentration of Fe is between about 0.2 and about 20 weight%, and especially between about 0.5 and about 10 weight%, based on the catalyst weight (metals + support).

Further details on the above components can be found from various of the previously cited references. Specific reference may be had, for example, to US2208598, US2257814, US2762835, US3322815, US5151543, US5296628, US5512697, US5527946 and WO99/47492.

Any additive comprising one or more of the above-mentioned compounds that can effect selectivity improvement is useful in the present invention.

The term "improvement" is referred to as enhanced selectivity to aminonitrile product at conversions greater than about 70%, preferably conversions greater than about 80%, and especially conversions greater than about 90%, as compared to the selectivity without the use of the additive of this invention. An "effective amount" of the additive is amount required to achieve the aforementioned enhanced selectivity and/or an improved overall yield of aminonitrile, as compared to without the use of the additive.

In preferred embodiments, the additive consists essentially of one or more of the above-mentioned compounds.

The additive is present during the contacting in any quantity that can improve the selective hydrogenation of a dinitrile to its corresponding aminonitrile (e.g., an effective amount).

For carbon monoxide, the preferred ratio of carbon monoxide to the catalyst is in the range of from 0.001:1 to 10:1, preferably 0.005:1 to 5:1, and especially 0.01:1 to 2:1, mmoles CO/g catalyst.

The catalyst and additive can be separately introduced into contact with a dinitrile; however, it is preferred that the catalyst, whether it is in its metal form or in an alloy or solid solution or on an inorganic support, is pretreated by contacting with the additive. This may be done in a solvent such as, for example, an alcohol, ether, ester, ammonia or combinations of two or more thereof. Further preferably the precontacting is also carried out in a hydrogen-containing fluid such as described above. Contacting of the catalyst and additive produces a pretreated catalyst. The pretreated catalyst can be washed with a solvent disclosed above, preferably under anaerobic condition to produce an additive-treated catalyst.

The contacting of the catalyst and additive can be carried out under any conditions effective to produce an additive-treated catalyst that can improve selective hydrogenation of a dinitrile or the selectivity to an aminonitrile. Generally, the entire process for producing the additive-treated catalyst can be carried out by contacting a catalyst with an additive disclosed above at a temperature in the range of from about 20°C to about 150°C, preferably about 30°C to about 100°C, under the same general pressures as described above, for about 5 seconds to about 25 hours.

For carbon monoxide, the preferred ratio of additive to catalyst in the pre-treating procedure generally ranges from 0.001:1 to 10:1, preferably from 0.005:1 to about 5:1, and more preferably from 0.01:1 to 2:1, mmoles CO/g catalyst.

The partial hydrogenation process of the present invention can be carried out at a temperature in the range of from about 25 to about 150°C, preferably about 40 to about 100°C, most preferably about 60 to about 80°C, at a total pressure generally in the range of about 50 to about 2000 psig (about 0.45 to about 13.89 MPa), with from about 200 to about 1000 psig (about 1.48 to about 7.00 MPa) preferred, for a time period generally in the range of from about 15 minutes to about 25 hours, preferably about 1 hour to about 10 hours.

The process of the invention can be operated batch wise or continuously in an appropriate reactor. Stirring or agitation of the reaction mixture can be accomplished in a variety of ways known to those skilled in the art. The partial hydrogenation of the starting dinitrile to its corresponding aminonitrile with high selectivity at high conversions of the dinitrile makes this process efficient and useful.

Further general and specific process details can be found from various of the previously cited references. Specific reference may be had, for example, to US2208598, US2257814, US2762835, US3322815, US5151543, US5296628, US5512697, US5527946 and WO99/47492.

The following examples further illustrate the process of the invention and are not to be construed to unduly limit the scope of the invention.

The meaning of terms used in the Examples is defined as follows:

Yield of aminonitrile is the measured concentration of aminonitrile divided by the starting concentration of dinitrile.

Conversion of the dinitrile is the difference between the starting and the instant concentration of dinitrile, divided by the starting concentration of dinitrile.

Selectivity to aminonitrile is the measured yield of aminonitrile divided by conversion of the dinitrile at that instance.

Where the use of hydrocyanic acid (hydrogen cyanide) is indicated, it was used as a condensed liquid measured in pre-chilled equipment to minimize evaporative losses.
COMPARATIVE EXAMPLE 1 - A sponge Ni catalyst (1.2 g) promoted with Fe and Cr (Activated Metals, A4000, without any further additives) was added to a 50 cc autoclave together with 3.2 g adiponitrile (ADN) and 35cc of liquid ammonia to form a mixture. Hydrogen was introduced to the autoclave and the ADN was hydrogenated at 60°C under the total pressure of 1045 psig (7.31 MPa) at ca. 1500 rpm. Total conversion of ADN was reached within 30 minutes on stream. The maximum yield of aminocapronitrile was 57% at 90% ADN conversion for a selectivitiy of 63%.
COMPARATIVE EXAMPLE 2 - To a 300 cc autoclave, was charged 7.7g Raney® Co (obtained from W.R. Grace Co., catalog number 2724), 0.77 g water, 26 g ADN, and 139 g liquid ammonia. The content was hydrogenated at 70°C, under the total pressure of 1000 psig (7.00 MPa) at 1000 rpm. Total conversion of ADN was reached within 40 minutes on stream. The maximum yield of aminocapronitrile was 58% at 90% ADN conversion for a selectivity of 64%.
COMPARATIVE EXAMPLE 3 - To a 50 cc autoclave, was charged 1.2g of a 5% rhodium on alumina catalyst (obtained from Engelhard), 3.2 g ADN, and 35 ml liquid ammonia. The content was hydrogenated at 80°C, under the total pressure of 1060 psig (7.41 MPa), at 1500 rpm. Total conversion of AND was reached within 30 minutes on stream. The maximum yield of aminocapronitrile was 3% at 96% ADN conversion, with the major product being hexamethylene diamine.
EXAMPLE 1 - 10.0 g of sponge Ni catalyst (Degussa BLM 112W) was charged into a 50cc autoclave, together with 1.0 ml of gaseous carbon monoxide under 60 psi of pressure (0.018 mmoles CO per g catalyst). Subsequently, 35 ml of liquid ammonia was added, and the mixture was heated to 80°C with stirring. The pressure was adjusted to 1000 psig (7.00 Mpa) with hydrogen, then kept under such conditions for 2.5 hrs. After cooling, the pressure was released and the sample was transferred to a dry box, washed with deaerated methanol, and stored under anaerobic conditions. 1.2 g of the sponge Ni catalyst, pretreated with carbon monoxide as described above, was charged into a 50cc autoclave together with 3.2 g of ADN and 35 ml of liquid ammonia, heated to 60°C, and reacted with hydrogen at a total pressure of 1000 psig (7.00 MPa). After 75 minutes, the yield of 6-aminocapronitrile reached ca. 72% at 94% ADN conversion for a selectivity of 77%.
EXAMPLE 2 - 1.2 g of the sponge Ni catalyst, pretreated with carbon monoxide, as described in Example 1, was charged into a 50cc autoclave together with 3.2 g of ADN and 35 ml of liquid ammonia, heated to 40°C, and reacted with hydrogen at a total pressure of 1000 psig (7.00 MPa). After 180 minutes, the yield of 6-aminocapronitrile reached ca. 72% at 93% ADN conversion for a selectivity of 77%.
EXAMPLE 3 - A 50cc autoclave was charged with 3.2 g of ADN, 1.2 g of Raney® Ni, 0.25 g of NaOH, 0.25 g of H₂O, 35 ml of MeOH and 6.42 ml of gaseous carbon monoxide under 40 psi pressure (0.077 mmoles CO per g catalyst). The mixture was heated to 70°C, then brought in contact with hydrogen for a total pressure of 500 psig (3.55 MPa), and run for 5 hours. After 4.5 hours, the yield of 6-aminocapronitrile reached ca. 63% at 78% ADN conversion for a selectivity of 81%. EXAMPLE 4 - 10.0 g of Raney® Co (W.R. Grace) was charged into a 50cc autoclave, together with 2.5 ml of gaseous carbon monoxide under 60 psi of pressure (0.045 mmoles CO per g catalyst). Subsequently, 35 ml of liquid ammonia was added, and the mixture was heated to 80°C with stirring. The pressure was adjusted to 1000 psig (7.00 Mpa) with hydrogen, then kept under such conditions for 2.5 hrs. After cooling, the pressure was released and the sample was transferred to a dry box, washed with deaerated methanol, and stored under anaerobic conditions. 1.2 g of the Raney® Co catalyst, pretreated with carbon monoxide as described above, was charged into a 50cc autoclave together with 3.2 g of ADN and 35 ml of liquid ammonia, heated to 40°C, and reacted with hydrogen at a total pressure of 1000 psig (7.00 MPa). After 160 minutes, the yield of 6-aminocapronitrile reached ca. 71% at 89% ADN conversion for a selectivity of 80%.
EXAMPLE 5 - 10.0 g of Raney® Co (W.R. Grace) was charged into a 50cc autoclave together with 5.0 ml of gaseous carbon monoxide under 80 psi of pressure (0.12 mmoles CO per g catalyst). Subsequently, 35 ml of liquid ammonia was added, and the mixture was heated to 80°C with stirring. The pressure was adjusted to 1000 psig (7.00 Mpa) with hydrogen, then kept under such conditions for 2.5 hrs. After cooling, the pressure was released and the sample was transferred to a dry box, washed with deaerated methanol, and stored under anaerobic conditions. 1.2 g of the Raney® Co catalyst, pretreated with carbon monoxide as described above, was charged into a 50cc autoclave together with 3.2 g of ADN and 35 ml of liquid ammonia, heated to 80°C, and reacted with hydrogen at a total pressure of 1000 psig (7.00 MPa). After 5 hours, the yield of 6-aminocapronitrile reached ca. 74% at 94% ADN conversion for a selectivity of 79%.

## Claims

1. A process for the partial hydrogenation of a dinitrile to an aminonitrile, comprising the step of contacting the dinitrile with a hydrogen-containing fluid in the presence of (a) a solvent comprising liquid ammonia, an alcohol, or both; (b) a hydrogenation catalyst; and (c) an additive for improving the yield of and/or selectivity to the aminonitrile, **characterized in that** the dinitrile has the general formula R(CN)₂, wherein R is an alkylene group, and the additive is carbon monoxide.

2. The process of claim 1, **characterized in that** the dinitrile is selected from the group consisting of adiponitrile, methylglutaronitrile and alpha,omega-dodecanedinitrile.

3. The process of claim 1, **characterized in that** the solvent is liquid ammonia.

4. The process of claim 1, **characterized in that** the hydrogenation catalyst comprises a transition metal selected from the group consisting of iron, cobalt, nickel, rhodium and combinations thereof.

5. The process of claim 4, **characterized in that** the hydrogenation catalyst is in sponge metal form.

6. The process of claim 4, **characterized in that** the catalytic metal is supported on an inorganic support.

7. The process of claim 1, **characterized in that** the hydrogenation catalyst is precontacted with the additive.

8. The process of any one of claims 1-7, **characterized in that** the ratio of carbon monoxide to hydrogenation catalyst is in the range of from 0.001:1 to 10:1 mmoles CO/g catalyst.

## Patentansprüche

1. Verfahren für die partielle Hydrierung eines Dinitrils zu einem Aminonitril, umfassend den Schritt des Kontaktierens des Dinitrils mit einer Wasserstoff enthaltenden Flüssigkeit in Gegenwart von (a) einem Lösemittel, das flüssiges Ammoniak aufweist, einen Alkohol oder Beides; (b) einen Hydrierungskatalysator; sowie (c) einem Additiv zur Verbesserung der Ausbeute und/oder Selektivität des Aminonitrils,
**dadurch gekennzeichnet, dass** das Nitril die allgemeine Formel R(CN)₂ hat, worin R eine Alkylen-Gruppe ist und das Additiv Kohlenmonoxid ist.

2. Verfahren nach Anspruch 1, **dadurch gekennzeichnet, dass** das Nitril ausgewählt ist aus der Gruppe, bestehend aus Adiponitril, Methylglutaronitril und alpha,omega-Dodecandinitril.

3. Verfahren nach Anspruch 1, **dadurch gekennzeichnet, dass** das Lösemittel flüssiges Ammoniak ist.

4. Verfahren nach Anspruch 1, **dadurch gekennzeichnet, dass** der Hydrierungskatalysator ein Übergangsmetall aufweist, das ausgewählt ist aus der Gruppe, bestehend aus Eisen, Kobalt, Nickel, Rhodium und Kombinationen davon.

5. Verfahren nach Anspruch 4, **dadurch gekennzeichnet, dass** der Hydrierungskatalysator in Form eines Metallschwammes vorliegt.

6. Verfahren nach Anspruch 4, **dadurch gekennzeichnet, dass** das katalytische Metall von einem anorganischen Träger gehalten wird.

7. Verfahren nach Anspruch 1, **dadurch gekennzeichnet, dass** der Hydrierungskatalysator mit dem Additiv vorkontaktiert ist.

8. Verfahren nach einem der Ansprüche 1 bis 7, **dadurch gekennzeichnet, dass** das Verhältnis von Kohlenmonoxid zum Hydrierungskatalysator im Bereich von 0,001:1 bis 10:1 mm CO/g Katalysator liegt.

## Revendications

1. Procédé pour l'hydrogénation partielle d'un dinitrile en un aminonitrile, comprenant l'étape de mise en contact du dinitrile avec un fluide contenant de l'hydrogène en présence (a) d'un solvant comprenant de l'ammoniaque liquide, un alcool ou les deux; (b) d'un catalyseur d'hydrogénation; et (c) d'un additif pour améliorer le rendement de et/ou la sélectivité en aminonitrile, **caractérisé en ce que** le dinitrile présente la formule générale R(CN)₂, dans laquelle R est un groupe alkylène et l'additif est du monoxyde de carbone.

2. Procédé suivant la revendication 1, **caractérisé en ce que** le dinitrile est choisi dans le groupe constitué d'adiponitrile, de méthylglutaronitrile et d'alpha,oméga-dodécanedinitrile.

3. Procédé suivant la revendication 1, **caractérisé en ce que** le solvant est de l'ammoniaque liquide.

4. Procédé suivant la revendication 1, **caractérisé en ce que** le catalyseur d'hydrogénation comprend un métal de transition choisi dans le groupe constitué de fer, de cobalt, de nickel, de rhodium et de combinaisons de ceux-ci.

5. Procédé suivant la revendication 4, **caractérisé en ce que** le catalyseur d'hydrogénation est sous une forme de métal éponge.

6. Procédé suivant la revendication 4, **caractérisé en ce que** le métal catalytique est supporté sur un support inorganique.

7. Procédé suivant la revendication 1, **caractérisé en ce que** le catalyseur d'hydrogénation est mis en contact au préalable avec l'additif.

8. Procédé suivant l'une quelconque des revendications 1-7, **caractérisé en ce que** le rapport du monoxyde de carbone sur le catalyseur d'hydrogénation est dans l'intervalle de 0,001:1 à 10:1 mmoles de CO/g de catalyseur.
